# EUROPEAN PATENT APPLICATION

(11) **EP 3 342 407 A1**
(43) Date of publication of application: **04.07.2018**
(21) Application number: 17150176.0
(22) Date of filing: 03.01.2017
(51) Int. Cl.: A61K 31/255, A61K 31/421, A61K 31/427, A61P 13/08

(54) **IL-8 INIHIBITORS FOR USE IN THE TREATMENT OF SOME UROLOGICAL DISORDERS**

(71) Applicant: Dompé farmaceutici S.p.A., 20122 Milan (IT)
(72) Inventor: ALLEGRETTI, Marcello, I-67100 L'Aquila (AQ) (IT); ARAMINI, Andrea, I-67100 L'Aquila (AQ) (IT); CESTA, Maria, Candida, I-67100 L'Aquila (AQ) (IT); BIANCHINI, Gianluca, I-67100 L'Aquila (AQ) (IT); BRANDOLINI, Laura, I-67100 L'Aquila (AQ) (IT)
(74) Representative: Viganò, Elena

(57) **Abstract**

The present invention relates to IL-8 inhibitor compounds for use in the treatment of chronic prostatitis/chronic pelvic pain syndrome (CP/CPPS) and benign prostatic hyperplasia.

## Description

### Technical field

The present invention relates to IL-8 inhibitor compounds for use in the treatment of chronic prostatitis/chronic pelvic pain syndrome (CP/CPPS) and benign prostatic hyperplasia.

### Background Art

Prostatitis is inflammation of the prostate gland, the walnut-sized gland located below a man's bladder. The prostate gland secretes fluid that, along with sperm, forms semen. There are different types of prostatitis, one of which is chronic prostatitis/chronic pelvic pain syndrome (CP/CPPS). This is the most common type. Young and middle-aged men are more likely to develop CP/CPPS, but it can happen at any age. CP/CPPS may be classified as inflammatory or non-inflammatory, based on the presence or absence of leukocytes in prostatic secretions, urine or semen. In inflammatory cases, urine, semen, and fluid secreted by the prostate contain infection-fighting cells, but these fluids don't contain bacteria.

Chronic prostatitis /chronic pelvic pain syndrome (CP/CPPS) is the most common urological disorder in men aged minor of 50 years, but its causes remain unexplained. This disease is poorly understood and is a highly prevalent condition of men that causes substantial morbidity. It's characterized by genital or pelvic pain in the absence of demonstrable urinary or genital tract infections, and is associated with urinary symptoms and sexual dysfunction (Luzzi GA, J Eur Acad Dermatol Venereol 2002, 16: 253). CP/CPPS is diagnosed in about 95% of patients with prostatitis syndrome (Brunner H et al, J Infect Dis 1983; 147:807; Pontari MA et al J Urol 2004; 172: 839).

Evidence is accumulating for a role of pro-inflammatory and anti-inflammatory cytokines in the development of the condition.

Benign prostatic hyperplasia is a common age-related proliferative abnormality of the human prostate. It's a chronic inflammatory disease of the urinary tract that affects the periurethral region, inducing glandular and stromal nodules within the transition zone, resulting in urinary obstruction (Lee KL et al. J Urol 2004; 172: 1784).

Benign prostatic hyperplasia (BPH) frequently has a significant detrimental impact on a patient's quality of life. If the disease is left untreated, it may progress in severity, leading to recurrent bladder infections, bladder calculi, and acute urinary retention (AUR), necessitating surgical treatment. About 14% of men aged 40 to 50 years have BPH and this percentage increases to 43% for men >60 years old. BPH has been shown to be nearly as prevalent as hypertension and diabetes among patients seeking treatment for erectile dysfunction. The effects of BPH on quality of life include lack of sleep, anxiety, reduced mobility, interference with leisure activities and usual daily activities, and a compromised sense of well-being. Three symptoms are associated with an increased risk of AUR in men with BPH: a reduction in the force of the urinary stream, a sensation of incomplete bladder emptying, and an enlarged prostate gland on digital rectal examination (Kirby RS et al, Urology, 2000; 56:3).

A wide variety of pharmacologic and nonpharmacologic therapies have been studied, but most have limited efficacy (Strauss AC et al, Nat Rev Urol, 2010; 7:127). Long term antibiotics were the mainstay of CP/CPPS treatment, although it's generally accepted that less than 10% of symptomatic patients have culturable bacteria in the urinary tract. Patients with localized inflammation of the prostate benefit of antinflammatory therapy based on Cyclooxygenase (COX) inhibitors, while in presence of autoimmune mechanisms, the use of corticosteroid therapy is an attractive option, such as the combination of prednisone with levofloxacin. In the most of cases an improvement of CP/CPPS symptoms was observed. Other agents such as alpha-blockers and 5-alpha reductase inhibitors are commonly used to treat patients with BPH, because able to inhibit overactivation of bladder smooth muscle and increase urine flow and, more recently have been implicated in blocking proliferation and inducing prostatic apoptosis (Yun AJ et al, Med Hypotheses, 2006; 67:392; Anglin IE et al, Prostate Cancer Prostatic Dis, 2002; 5:88). Many other therapies have been studied in CP/CPPS and BPH patients, most with variable results.

Chemokines constitute a large family of chemotactic cytokines that exert their action via an interaction with receptors belonging to the seven Transmembrane G Protein Coupled Receptor (7TM-GPCRs) family. The chemokine system is crucial for the regulation and the control of the basal homeostatic and inflammatory leukocyte movement. Many cell types, besides the hematopoietic cells, express chemokine receptors; they include endothelia, smooth muscle cells, stromal cells, neurons and epithelial cells.

Among chemotactic factors, Interleukin-8 (IL-8; CXCL8) is considered a major mediator of PMN (Polymorphonuclear Neutrophils) recruitment and involved in several pathologies including psoriasis, rheumatoid arthritis, chronic obstructive pulmonary disease and reperfusion injury in transplanted organ (Griffin et al, Arch Dermatol 1988, 124: 216; Fincham et al, J Immunol 1988, 140: 4294; Takematsu et al, Arch Dermatol 1993, 129: 74; Liu et al, 1997, 100:1256; Jeffery, Thorax 1998, 53: 129; Pesci et al, Eur Respir J. 1998, 12: 380; Lafer et al, Br J Pharmacol. 1991, 103: 1153; Romson et al, Circulation 1993, 67: 1016; Welbourn et al, Br J Surg. 1991, 78: 651; Sekido et al, Nature 1993, 365, 654).

The biological activity of Interleukin-8 is mediated by the interaction with the CXCR1 and CXCR2 receptors belonging to the 7TM-GPCR family that are expressed on the surface of human PMNs. The two human receptors are highly homologous (77% amino acid identity), and the greatest diversity is focused at three regions: the N terminus (the ligand-binding region), the fourth transmembrane domain and the C terminus (Lee et al, J Biol Chem 1992, 267: 16283).

While human CXCR1 is quite selective, binding with high affinity only two chemokines, IL-6 and IL-8, and showing a much higher affinity for IL-8 [Wolf et al, Eur J Immunol 1998, 28: 164], human CXCR2 a is a more promiscuous receptor, binding a number of different cytokines and chemokines in addition to the two above, such as for example IL-1, IL-2, IL-3, IL-5, and IL-7 (Chapman et al., Pharmacology & Therapeutics 121 (2009) 55). Therefore, CXCR2 mediates the activity of a number of different mediators.

For both receptors, following activation the responses are regulated by phosphorylation at specific residues of the C-terminus that causes the association with an heterotrimeric G-protein complex which dissociates into its subunits to stimulate effector molecules and, thereby, causes activation of phospholipase C, resulting in the generation of the intracellular messenger diacylglycerol and inositol 1,4,5-triphosphate.

Following CXCL8 activation, CXCR1 and CXCR2 become desensitized and downregulated by internalization of the receptor (Richardson et al, J Biol Chem 1998; 273: 23830 Richardson et al, J Immunol. 2003, 170: 2904; Premont et al, Annu Rev Physiol 2007, 69: 511).

CXCR1 and CXCR2 are phosphorylated via two main mechanisms: a protein kinase C-dependent mechanism and a GRK (GPCR kinase)-dependent mechanism. For example, the C-terminal tail phosphorylation of CXCR1 is required for processes such as chemotaxis and receptor internalization. It has been shown that the two receptors, CXCR1 and CXCR2, are coupled to different intracellular pathways through the interaction with distinct GRK isoforms. In particular, CXCR1 predominantly couples to GRK2, whereas CXCR2 interacts with GRK6 to negatively regulate receptor sensitization and trafficking, thus affecting cell signaling and angiogenesis (Raghuwanshi et al, J Immunol 2012, 189: 2824). Upon IL-8 activation, CXCR1 slowly internalizes (45% after 60 min) but recovers rapidly (100% after 90 min), whereas CXCR2 internalizes rapidly (95% after 10 min) but recovers slowly (35% after 90 min) at the cell surface (Richardson et al, J Immunol 2003, 170: 2904; Chuntharapai et al, J Immunol 1995, 1995, 155: 2587). This distinction appears critical in the ability of the two receptors to activate specific leukocyte responses, including respiratory burst and postendocytic signals. Despite evidence that the two receptors signal through similar G proteins, there are marked differences in the activation of signaling cascade between CXCR1 and CXCR2, which identifies diverse functions. For example, inhibition of CXCR1 but non CXCR2 causes a decrease in superoxide anion production by PMNs, indicating a pivotal role of CXCR1 in oxidative burst (Jones et al, J Biol Chem 1997, 272: 16166; Jones et al, PNAS USA 1996, 93: 6682). In addition, CXCR1 activates PLD1 (phospholipase D1), whereas CXCR2 mediates PLD2 (phospholipase D2) activation that catalyzes the hydrolysis of phosphatidylcholine to phosphatidic acid and choline (Palicz et al, J Biol Chem 2001, 276: 3090).

A number of studies have investigated the role of IL-8 in urological disorders.

WO2010/078403 discloses that a number of cytokines, chemokines and growth factors, including IL-8, are increased in the urine of patients affected by urological disorders and hypothesizes that the identification of elevated concentrations of these proteins in the urine can be used as a diagnostic tool. Multiple proteins are identified in the document as potential biomarkers of urological pathologies, all of these being well known inflammation mediators.

Jiang et al disclose increased levels of pro-inflammatory cytokines and chemokine including IL-1β, IL-6, TNF-α, and IL-8, as well as serum C-reactive protein (CRP), nerve growth factor (NGF) in patients with IC/PBS compared to controls (PlosOne 2013, 10: e76779).The above documents teach that IC/PBS is associated with the presence in the urine or serum of the patients of a number of mediators of inflammation, including IL-8, but do not provide any teaching as regards the specific role of each of these mediators in the onset and progression of the disease. Furthermore, the documents lack any information on the effect of inhibition of the identified potential markers on the onset and/or progression of urological disorder.

Some publications disclose data that suggests that IL-8 and CXCR1 have an important role in the maintenance of the health of the urinary tract.

In fact, it has been demonstrated that IL-8 exerts a protective effect of on the urothelium and that lower IL-8 expression levels in the urinary bladder may contribute to pathophysiology of different urological disorders (Tseng-Rogenski et al, Am J Physiol Renal Physiol 2009, 297: F816-F821).

As regards IL-8, the above described documents suggest that this chemokine and, in particular, its activity through CXCR1 receptor, plays a pivotal role in normal urothelial cell survival and that a decreased level of expression of IL-8 or CXCR1 in the urinary bladder contributes to the pathophysiology of urinary disorders.

### Summary of Invention

Surprisingly, the Applicant has now found that, in contrast with the teaching of the prior art, inhibitors of IL-8, are useful in the treatment and/or prevention of chronic prostatitis/chronic pelvic pain syndrome (CP/CPPS) and benign prostatic hyperplasia. Accordingly, the first object of the present invention is an IL-8 inhibitor, preferably an antibody or small molecule, for use in the treatment and/or prevention of chronic prostatitis/chronic pelvic pain syndrome (CP/CPPS) and benign prostatic hyperplasia.

The second object of the present invention is the use of said IL-8 inhibitor as defined above, for the preparation of a medicament for the treatment and/or prevention chronic prostatitis/chronic pelvic pain syndrome (CP/CPPS) and benign prostatic hyperplasia.

The third object of the present invention is a method for the treatment and/or prevention of chronic prostatitis/chronic pelvic pain syndrome (CP/CPPS) and benign prostatic hyperplasia, in a subject comprising the step of administering to the subject in need thereof a therapeutically effective amount of said IL-8 inhibitor. The fourth object of the present invention is a pharmaceutical formulation for use in the treatment and/or prevention of chronic prostatitis/chronic pelvic pain syndrome (CP/CPPS) and benign prostatic hyperplasia comprising (a) an IL-8 inhibitor as defined above and (b) one or more further pharmaceutically active compounds.

The fifth object of the present invention is a kit for use in the treatment and/or prevention of chronic prostatitis/chronic pelvic pain syndrome (CP/CPPS) and benign prostatic hyperplasia, comprising an IL-8 inhibitor as defined above and one or more pharmaceutically active compounds for simultaneous, separate or sequential use.

### Figure description

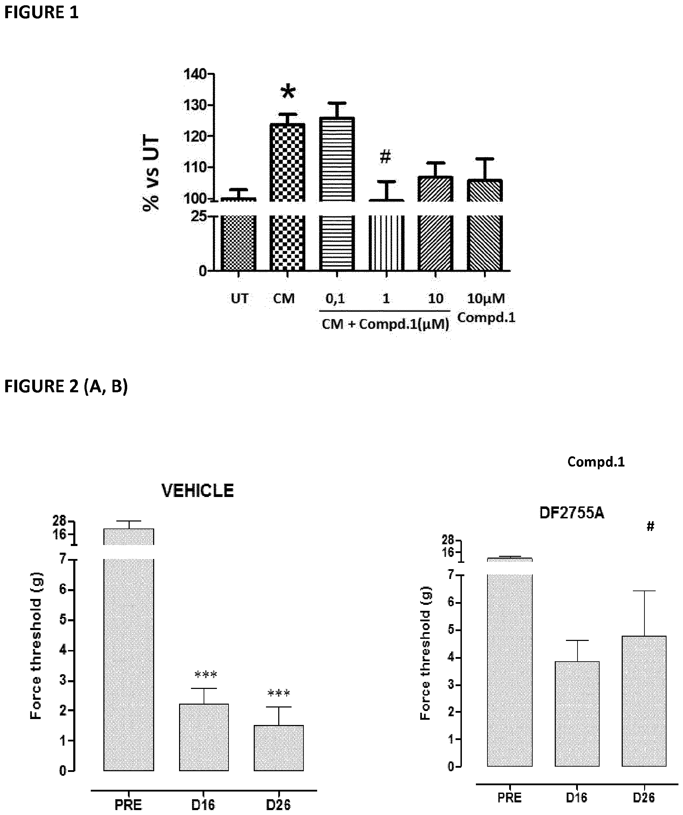

**Figure 1** shows the oxidative stress measurement in RWPE-1 cells treated with Compd.1 at the dosages of 0.1, 1 and 10 µM, for 24 hours and then fed with conditioned medium (CM), as described in Example 1. The graph reports the levels of total ROS and RNS production in the different treatment conditions. Data are presented as mean of the percentages vs untreated group (% vs UT) ± SEM. *p<0.05 vs UT; # p<0.05 vs CM (one way ANOVA with Bonferroni post hoc test).
**Figure 2** shows the effect of oral administration of vehicle (A) and Compound 1 (Compd.1, B), administered at a dosage of 20mg/kg, on the abdomen mechanical threshold, expressed in grams, as described in Example 2. The graphs report values measured at three different time-points: pre-immune values (PRE); 16 days post immunization values (D16); 26 days post immunization values (D26). Data show the mean ± SE. ***p<0.001 vs PRE (non-parametric Kruskal-Wallis test followed by Dunn's multiple comparison post-test); # p<0.05 vs Vehicle (unpaired t test with Welch's correction).

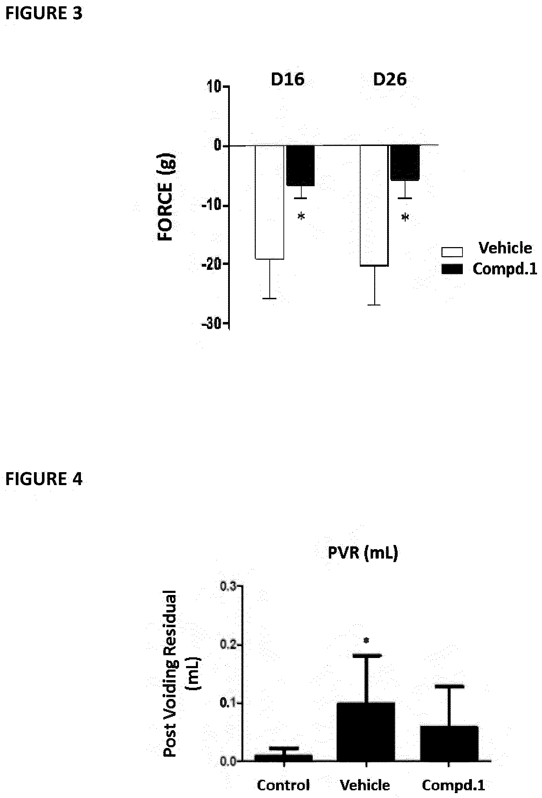

**Figure 3** shows the effect of oral administration of vehicle and Compound 1 (Compd.1), administered at a dosage of 20mg/kg, on the abdomen mechanical threshold, expressed in grams of force, at 16 days post immunization (D16) and at 26 days post immunization (D26), as described in Example 2. For each animal, the D16 and D26 post-immune withdrawal threshold value has been subtracted from the respective pre-immune baseline threshold value. Data are represented as mean ± SE. *p<0.05 vs Vehicle (unpaired Student's T test).
**Figure 4** shows the effect of oral administration of vehicle and Compound 1 (Compd.1), administered at the dosage of 20 mg/kg, on the post void residual volume (PVR) expressed in mL, as described in Example 3. Data represent the post voiding residual volumes of control, vehicle and Compd.1 groups. Data are expressed as mean ± SD. *p<0.05 vs Control (one way ANOVA with Tukey's multiple comparison post-test).

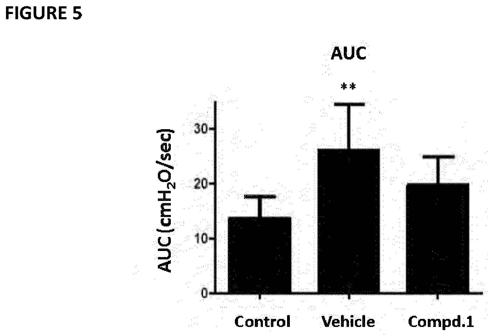

**Figure 5** shows the effect of oral administration of vehicle and Compound 1 (Compd.1), administered at the dosage of 20 mg/kg, on the quantification of detrusor over activity measured as area under pressure/time curve (AUC), expressed in cmH₂O/sec as described in Example 3. Data represent the AUC values of control, vehicle and Compd.1. Data are expressed as mean ± SD. **p<0.01 vs Control (one way ANOVA with Tukey's multiple comparison posttest).

### Detailed description of the invention

As will be disclosed in details in the Experimental section, small molecules that inhibit the activity of IL-8 have surprisingly shown *in vivo* therapeutic efficacy in experimental animal models of chronic prostatitis and benign prostatic hyperplasia. Accordingly, a first object of the present invention is an IL-8 inhibitor for use in the treatment and/or prevention of chronic prostatitis/chronic pelvic pain syndrome (CP/CPPS) and benign prostatic hyperplasia.

The term "IL-8-inhibitor" according to the present application refers to any compound able to inhibit, partially or totally, the biological activity of IL-8. Such a compound can act by decreasing the expression or activity of IL-8 or by inhibiting the triggering of the intracellular signaling by activation of the IL-8 receptors. In the latter case, such compound is preferably either an allosteric inhibitor or an antagonist of CXCR1 or of both CXCR1 and CXCR2 receptors. Preferably, said IL-8 inhibitor is able to inhibit chemotaxis induced by IL-8 in PMNs with a concentration in the low microMolar or nanoMolar range.

According to preferred embodiments of the invention, said IL-8 inhibitor is a CXCR1 inhibitor, more preferably it is a dual CXCR1/CXCR2 inhibitor.

According to further preferred embodiments of the invention, also in combination with the preceding embodiments, said IL-8 inhibitor is an antibody, a peptide or small molecule inhibitor.

To date, several IL-8 inhibitors, such as small molecules, peptides and antibodies, have been disclosed, many of which are currently under undergoing clinical trials or are used in therapy. i. e. SK&F 83589, SB225002 (Jie Jack, Expert Opinion Ther. Patents, 2001, 11(12), p. 1905-1910), C(4)-alkyl substituited furanyl cyclobutenediones (Chao J. et al., Bioorganic & Medicinal Chemistry Letters 17, 2007, p. 3778-3783) and different small molecules from GlaxoSmithKline, Astra Zeneca, Pfizer and Schering-Plough (Busch-Petersen J. Current Topics in Medicinal Chemistry, 2006, 6, p. 1345-135 and Allegretti et al, Immunology Letters 2012, Vol. 145, p. 68-78).

Among small molecules inhibitors of IL-8, preferred compounds according to the invention are 1,3-thiazol-2-ylaminophenylpropionic acid derivatives, 2-phenyl-propionic acid derivatives and their pharmaceutically acceptable salts.

According to one preferred embodiment, said 2-pheny-propionic acid derivatives are compounds of formula (I) 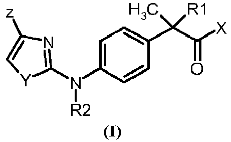 wherein
R1 is hydrogen;
X is OH;
R2 is hydrogen or linear C₁-C₄ alkyl;
Y is a heteroatom selected from S, O and N;
Z is selected from linear or branched C₁-C₄ alkyl, linear or branched C₁-C₄ alkoxy, halo C₁-C₃ alkyl and halo C₁-C₃ alkoxy.

Preferably, Z is CF₃.

More preferably, said compounds of formula (I) have the chiral carbon atom of the phenylproprionic group in the S configuration.

Particularly preferred compounds of formula (I) according to the inventions are selected from (R,S)-2-(4-{[4-(trifluoromethyl)-1,3-thiazol-2-yl]amino}phenyl)propanoic acid or (2S)-2-(4-{[4-(trifluoromethyl)-1,3-thiazol-2-yl] amino} phenyl) propanoic acid and pharmaceutically acceptable salts thereof, preferably a sodium salt. The most preferred 2-aryl- propionic acid derivative according to the invention is the sodium salt of (2S)-2-(4-{[4-(trifluoromethyl)-1,3-thiazol-2-yl] amino} phenyl) propanoic acid (hereinbelow indicated as Compd.1) Compounds of formula (I) are disclosed in WO2010/031835 which also discloses their method of synthesis, their activity as IL-8 inhibitors as well as their use in the treatment of IL-8 dependent pathologies such as transient cerebral ischemia, bullous pemphigoid, rheumatoid arthritis, idiopathic fibrosis, glomerulonephritis and damages caused by ischemia and reperfusion. Compd.1 is also specifically disclosed therein and corresponds to compound (3a) of the document.

The present inventors have investigated the pharmacokinetic profile of Compd.1 and have found that this is particularly advantageous for a use in urinary disorders such as chronic prostatitis/chronic pelvic pain syndrome (CP/CPPS) and benign prostatic hyperplasia.

In fact, as it will be illustrated in the Experimental section, Compd. 1 shows a rapid absorption and reaches a maximum concentration (Cmax) in plasma.

According to another preferred embodiment, said 2-phenyl-propionic acid derivative is a compound of formula (II) 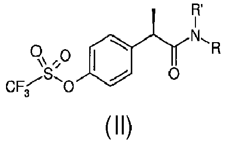 or a pharmaceutically acceptable salts thereof,
wherein
R' is hydrogen;
R is a residue of formula SO₂Ra wherein Ra is linear or branched C₁-C₄ alkyl or halo C₁-C₃ alkyl.

More preferably, said compounds of formula (II) have the chiral carbon atom of the phenylpropionic group in the R configuration.

Even more preferably, the said compound of formula (II) is R(-)-2-[(4'-trifluoromethanesulfonyloxy)phenyl]-N-methanesulfonyl propionamide or a pharmaceutically acceptable salt thereof, preferably a sodium salt. Most preferably said compound of formula (II) is the sodium salt of R(-)-2-[(4'-trifluoromethane sulfonyloxy)phenyl]-N-methanesulfonyl propionamide (hereinbelow indicated as Compd.2).

2-(R)-Phenyl-propionic acid derivative of formula (II) are disclosed in WO2005/090295; also their method of synthesis, their activity as IL-8 inhibitors as well as their use in the treatment of pathologies like psoriasis, ulcerative colitis, melanoma, chronic obstructive pulmonary diseases (COPD), bullous pemphigo, rheumatoid arthritis, idiopathic fibrosis, glomerulonephritis and damages caused by ischemia and reperfusion is disclosed therein.

Compd.2 is also specifically disclosed therein and corresponds to compound (1 a) of the above document. Compd.2 is a potent and selective dual CXCR1/CXCR2 non-competitive allosteric inhibitor (Bertini R. et al, Br J Pharmacol 2012, 165(2):436-54).

The second object of the present invention is the use of an IL-8 inhibitor as already defined above for the preparation of a medicament for the treatment and/or prevention of chronic prostatitis/chronic pelvic pain syndrome (CP/CPPS) and benign prostatic hyperplasia.

The third object of the present invention is a method for the treatment and/or prevention of chronic prostatitis/chronic pelvic pain syndrome (CP/CPPS) and benign prostatic hyperplasia in a subject comprising the step of administering to the subject in need thereof a therapeutically effective amount of an IL-8 inhibitor as already defined above.

As used herein, a "therapeutically effective amount" refers to an amount sufficient to achieve treatment or prevention of the disease. Determination of the effective amounts is well within the capability of those skilled in the art based upon the achievement of a desired effect. An effective amount will depend on factors including, but not limited to, the weight of a subject and/or the degree to which the disease or unwanted condition from which a subject suffers. The terms "treatment" and "prevention" as used herein refer to the eradication/amelioration or prevention/delay in onset, respectively, of the disorder being treated or of one or more of the symptoms associated thereof, notwithstanding the fact that the patient may still be afflicted with the underlying disorder.

The fourth object of the present invention is a pharmaceutical composition comprising an IL-8 inhibitor as defined above for use in the treatment and/or prevention of chronic prostatitis/chronic pelvic pain syndrome (CP/CPPS) and benign prostatic hyperplasia in association with pharmaceutically suitable excipients.

Preferably, said pharmaceutical composition further comprises at least one further pharmaceutically active compound.

The fifth object of the present invention is a product or kit comprising:
A) an IL-8 inhibitor as defined above for use in the treatment and/or prevention of chronic prostatitis/chronic pelvic pain syndrome (CP/CPPS) and benign prostatic hyperplasia or a pharmaceutical composition as defined above, and
B) at least one further pharmaceutically active compound A) and B) being two separate formulations for simultaneous, separate or sequential use.

According to one preferred embodiment of the fourth or fifth object of the invention, said further pharmaceutically active compound of said pharmaceutical composition or kit is an active compound useful for the prevention and treatment of chronic prostatitis/chronic pelvic pain syndrome (CP/CPPS) and benign prostatic hyperplasia.

Preferably, according to this embodiment, said further pharmaceutically active compound is selected from antibiotics, anti-inflammatory agents, alpha-blockers and 5-alpha reductase inhibitors.

In a preferred embodiment said antibiotics are selected from antibiotics ciprofloxacin, minocycline, levaquin, lomefloxacin, erithromycin, azithromycin, clarithromycin, quinolones ad macrolides, more preferably minocycline, lomefloxacin, erithromycin and ciprofloxacin, levaquin, lomefloxacin.

In a further preferred embodiment said anti-inflammatory agents are selected from NSAIDs such as ketoprofen, ibuprofen and nimesulide, or corticosteroids such as prednisolone, more preferably ketoprofen, nimesulide, and prednisolone. Preferably, said alpha-blockers are selected from doxazosin, terazosin, tamsulosin, and alfuzosin, more preferably doxazosin and terazosin.

Preferably, said 5-alpha reductase inhibitor is finasteride.

According to a further object, the pharmaceutical composition of the present invention for use in the treatment and/or prevention of chronic prostatitis/chronic pelvic pain syndrome (CP/CPPS) and benign prostatic hyperplasia is associated with non-pharmacological therapies, selected from Myofascial trigger point therapy and transurethral microwave thermotherapy (Chery nee King N, J Ass Chartered Physiotherapists Women's Health 2013, 112:41-4; Furuya R et al., Urology. 2007, 70:922-6.).

For the purpose of the present invention, the inhibitors of IL-8 according to the present invention are formulated in pharmaceutical compositions suitable for use by oral formulation, such as tablets, capsules, syrups, preferably in the form of controlled release formulations, or by parenteral administration, preferably in the form of sterile solutions suitable for intravenous or intramuscular administration. The pharmaceutical compositions can be prepared according to conventional methods, for example as disclosed in Remington, "The Science and Practice of Pharmacy", 21st ed. (Lippincott Williams and Wilkins).

Preferably, the amount of Compd.1 or its pharmaceutically acceptable salt in each of the above-mentioned administration forms will be such as to provide between 10 and 30 mg compound or salt/kg body weight, while the amount of Compd. 2 or its pharmaceutically acceptable salt will be such as to provide between 200 and 300 mg compound or salt/kg body weight. In any case, the regimen and amount of medicament to be administered will be determined by the physician according to the human pharmacokinetics.

The invention will be further illustrated in greater details in the following experimental section.

### Experimental section

### Example 1

### Characterization of Compound 1 on an in vitro inflammatory model of prostatitis.

In the aim of characterizing the Compound 1 on an in vitro inflammatory prostatitis model, human prostatic normal cells RWPE-1 (ATCC, lot number 61840713) are put in communication with activated macrophages; thus it induces an inflammatory micro-environment that enhances the production of reactive oxygen and nitrogen species (Debelec-Butuner et al, Mol. Carcinogen. 2014, 53:85-97).

Human U-937 monocytes (ATCC, lot number 61795631) seeded at 50.000 cells/ml were differentiated with phorbol acetate (16 mM) for 16 h. In order to stimulate cytokines production, lipopolysaccharide (LPS) was added (10ng/ml for 24h) and the conditioned medium (CM) collected.

After 24h in culture, RWPE-1 cells were treated with Compound 1 (0.1, 1 and 10 µM) and then exposed to CM from activated U-937. After 3 hours, reactive oxygen species (ROS) and Reactive Nitrogen species (RNS) were quantitatively measured by means of 2',7'-dichlorodihydrofluorescein diacetate (DCFH-DA) assay. After diffusion into the cell, DCFH-DA is deacetylated by cellular esterases to a non-fluorescent compound (DCFH), which is later oxidized by ROS and RNS into 2', 7' -dichlorofluorescein (DCF). DCF is a highly fluorescent compound which can be detected by fluorescence spectroscopy with maximum excitation and emission spectra of 495 nm and 529 nm respectively (Gomes et al, J. Biochem. Biophys. Methods 2005, 65:45-80).

The results clearly showed that CM significantly induced ROS and RNS production. Compound 1 when administered at 1µM completely reverted the CM effect; at the concentration of 10 µM, Compound 1 showed a more modest effect in comparison to the concentration 1µM, probably due to a slight detrimental effect of the compound alone at that dose (Figure 1).

### Example 2

### Effect of oral Compound 1 in experimental Autoimmune Chronic Prostatitis (EACP)

The experimental autoimmune chronic prostatitis (EACP) can be easily generated in rats by specific immunization with syngeneic rat prostate homogenate. As a consequence of the strong inflammatory reaction, pelvic pain is generated, as already described (Zhang et al, Scand J Immunol 2011, 73:546-553; Zhang et al, Prostate 2012, 72:90-99; Wang et al, Int Urol Nephrol 2015, 47:307-316; Rudick et al, Am J Physiol Regul Integr Comp Physiol 2008, 294 :R1268-1275).

A total of 36 Lewis male rats (250-270g) from Charles River Italy were used for the study, according to the regulations of the Animal Ethics Committee (IACUC n.631). The rats were maintained under standard laboratory conditions at 12:12 light/dark cycle with free access to food pellets and tap water.

Each animal was initially immunized with 2 mg of syngeneic prostate protein, diluted in saline, emulsified with complete Freund adjuvant containing 2 mg/ml of H37a mycobacterium (BD). Emulsion was produced by vortexing the mix for 15 min or till obtaining appropriate viscosity. A total volume of 0.2 mL in two sites was injected into each anesthetized rat at the dorsal tail base. A booster immunization was then repeated 13 days after the first injection.

Rats were randomly allocated to receive by oral gavage (1mg/kg) Compound1 (20mg/kg; n=12) or vehicle (saline alone; n=13) twice daily, 6 hours apart, encompassing 16 consecutive administrations plus 5 additional ones after a washout period (from day 1 to 16 and day 22 to 26).

Effect of pelvic pain was evaluated 16 and 26 days after immunization by testing mechanical allodynia with Von Frey test. Von Frey filaments of different gauges or stiffness were used to determine the threshold that elicits an abdomen withdrawal response. The mechanical withdrawal threshold was defined as the minimum gauge Von Frey filament that elicits a withdrawal reflex.

Quantification of mechanical allodynia demonstrated that EACP induced a significant reduction of pelvic withdrawal threshold at day 16 and 26 compared to the pre-immune status (Figure 2, A, vehicle); at the same time points, Compound1 partly decreased the mechanical hypersensitivity and allodynia reaching the statistical significance at day 26 (Figure 2, B), when compared to vehicle values.

In Von Frey test, vehicle group animals were stimulated by a force of 19g; rats treated with Compound1 responded to an applied force which was 2,8-fold significantly higher compared to the effective stimulus in condition of normal sensation (Figure 3).

### Example 3

### Effect of oral administration of Compound 1 on urodynamic parameters in rats with testosterone-induced prostate hypertrophy (TBPH)

The study was undertaken to test the effects of chronic oral administration of Compound 1 in rats with dysfunctional bladders in an experimental model of TBPH involving an estrogen-associated inflammation (Tatemichi et al, J Urol 2006, 176:1236-1241).

A total 26 Sprague-Dawley male rats (250-270g) were used for the study, according to the regulations of the Animal Ethics Committee (IACUC n.631). The rats were maintained under standard laboratory conditions at 12:12 light/dark cycle with free access to food pellets and tap water.

BPH was induced treating animals once a week for 4 weeks with intramuscular hormones corresponding to testosterone enanthate (Geymonat, 12.5 mg) + 17β-estradiol-velerate (SIGMA, 0.125 mg) in sesame oil. Control naïve animals (n=6) received sesame oil injection alone.

After first hormonal treatment, rats were randomly allocated to receive oral (1 ml/kg) Compound 1 (20mg/kg; n=10) or vehicle (n=9) by gavage, twice daily, 5 days a week.

Four weeks after initial treatment a bladder catheter was implanted, as already described (Gratzke et al, Eur Urol 2010, 57:1093-1100; Streng et al, Eur Urol 2008, 53:391-399). Cystometry was performed two days after the catheter implantation.

The conscious rats were placed in metabolic cages without restraint and the bladder catheters were connected via a T-tube to a pressure transducer (P23 DC; Statham Instruments Inc., Oxnard, CA, USA) and a microinjection pump (CMA 100; Carnegie Medicine AB, Solna, Sweden). Micturition volumes were recorded with a fluid collector connected to a force displacement transducer (FT 03 D, Grass Instrument Co., Quincy, MA, USA). Room-temperature saline was infused into the bladder continuously at a rate of 10 mLh-1. Pressures and micturition volumes were recorded continuously with Acq Knowledge 3.8.1 software and a MP100 data acquisition system (Biopac Syst. Inc. Santa Barbara, CA) connected to a Grass polygraph (Model 7E, Grass Instrument Co).

Testosterone treatment induced a significant changes in all bladder pressures, micturition (data not shown) and residual volumes (Figure 4). Compound 1 showed a numerical trend in reducing some of parameters considered, including the post void residual volume (PVR) and the quantification of the area under pressure/time curve (AUC, indicating detrusor over activity) as a possible results of lower exposure to inflammatory stimuli that act locally. (Figure 4 and Figure 5, respectively).

## Claims

1. A IL-8 inhibitor for use in the treatment and/or prevention of chronic prostatitis/chronic pelvic pain syndrome (CP/CPPS) and benign prostatic hyperplasia.

2. An IL-8 inhibitor for use according to claims 1, which is a CXCR1 inhibitor or a dual CXCR1 and CXCR2 inhibitor.

3. An IL8 inhibitor for use according to claim 1 or 2, selected from a small molecule, antibody or peptide.

4. An IL-8 inhibitor for use according to claims 1 to 3, having formula (I) 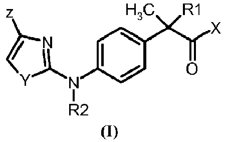 and pharmaceutically acceptable salts thereof,
wherein
R1 is hydrogen;
X is OH;
R2 is hydrogen or linear C₁-C₄ alkyl;
Y is a heteroatom selected from S, O and N;
Z is selected from linear or branched C₁-C₄ alkyl, linear or branched C₁-C₄ alkoxy, halo C₁-C₃ alkyl and halo C₁-C₃ alkoxy.

5. An IL-8 inhibitor for use according to claims 1 to 4, wherein said IL-8 inhibitor is selected from (R,S)-2-(4-{[4-(trifluoromethyl)-1,3-thiazol-2-yl]amino}phenyl)propanoic acid and (2S)-2-(4-{[4-(trifluoromethyl)-1,3-thiazol-2-yl] amino} phenyl) propanoic acid, preferably as sodium salt.

6. An IL-8 inhibitor for use according to claims 1 to 3, having formula (II) 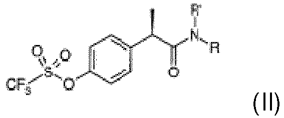 and pharmaceutically acceptable salts thereof,
wherein
R' is hydrogen;
R is a residue of formula SO₂Ra wherein Ra is linear or branched C₁-C₄ alkyl or halo C₁-C₃ alkyl.

7. An IL-8 inhibitor for use according to claims 1 to 3 and 6, wherein said IL-8 inhibitor is R(-)-2-[(4'-trifluoromethanesulfonyloxy)phenyl]-N-methanesulfonyl propionamide, preferably the sodium salt thereof.

8. A pharmaceutical composition comprising an IL-8 inhibitor for use according to any of the previous claims.

9. A pharmaceutical composition for use according to claim 8, further comprising at least one further pharmaceutically active compound.

10. A product or a kit for use according to any one of claims 1 to 7, comprising:
A) an IL-8 inhibitor according to any one of claims 1 to 7, or a pharmaceutical composition according to claim 8, and
B) at least one further pharmaceutically active compound A) and B) being two separate formulations for simultaneous, separate or sequential use.

11. A pharmaceutical composition according to claim 9 or a kit according to claim 10, wherein said further pharmaceutically active compound is an active compound useful for the prevention and treatment of chronic prostatitis/chronic pelvic pain syndrome (CP/CPPS) and benign prostatic hyperplasia.

12. A pharmaceutical composition or a kit according to claim 11, wherein said further pharmaceutically active compound is selected from antibiotics, anti-inflammatory agents, alpha-blockers and 5-alpha reductase inhibitors.
